# FASCICULE DE BREVET EUROPEEN

(11) **EP 0 852 132 B1**
(45) Date de publication et mention de la délivrance du brevet: **11.06.2003**
(21) Numéro de dépôt: 97403095.9
(22) Date de dépôt: 19.12.1997
(51) Int. Cl.: A61F 2/01

(54) **Filtre sanguin à perméabilité améliorée**
Blutfilter mit verbesserter Durchlässigkeit
Blood filter with improved permeability

(30) Priorité: 03.01.1997 FR 9700032
(43) Date de publication de la demande: 08.07.1998
(73) Titulaire: B. BRAUN CELSA (société anonyme), 86360 Chasseneuil du Poitou (FR)
(72) Inventeur: Chevillon, Gerard, 92120 Montrouge (FR); Nadal, Guy, 86000 Poitiers (FR)
(74) Mandataire: Lerner, François

(56) Documents cités:
- EP-A- 0 582 493
- EP-A- 0 655 228
- WO-A-96/17634
- FR-A- 2 718 950

## Description

L'invention concerne un filtre sanguin destiné à être positionné dans un vaisseau sanguin, pour y piéger des caillots de sang transportés par le flux sanguin.

On notera dès à présent que le filtre de l'invention peut être tant un filtre "définitif" (ou permanent), qu'un filtre "temporaire", c'est-à-dire un filtre implantable dans un vaisseau et qui peut y être laissé à demeure, ou bien en être retiré, après une certaine période d'implantation (typiquement considérée aujourd'hui comme de l'ordre d'une quinzaine de jours).

De façon conventionnelle, un filtre dit à implantation "définitive" est pourvu de moyens de fixation ou d'accrochage à la paroi du vaisseau dans lequel il est implanté.

Des exemples de tels filtres sont décrits dans US-A-5 059 205, US-A-5 133 733, ou encore US-A-5 344 427.

On connait toutefois également certains tels filtres sanguins pourvus de crochets de fixation à la paroi du vaisseau, qui sont malgré tout définis comme étant retirables ou repositionnables, du moins si la période pendant laquelle ils ont été positionnés en un endroit précis d'un vaisseau n'a pas été trop longue et que le développement cellulaire n'empêche pas en pratique de les déplacer.

US-A-5 324 304 présente un tel filtre dont la tête est équipée d'un crochet permettant d'attraper le filtre.

Toutefois, différents filtres dits "temporaires", c'est-à-dire retirables dans les conditions rappelées ci-avant, sont tels que le filtre en lui-même est raccordé à une longue tubulure porteuse souple (souvent un cathéter) qui serpente dans le corps du patient jusqu'à la zone de surface cutanée à partir de laquelle le filtre a été introduit dans le système vasculaire, cette tubulure allongée s'étendant même en général jusqu'à l'extérieur du corps du patient.

Des exemples de tels filtres sont décrits dans US-A-5 300 086, dans FR-A-2 713 081 ou encore dans US-A-4 969 891.

Parmi ces différents types de filtres, celui de l'invention concerne, structurellement, plus particulièrement un filtre présentant un axe et comprenant une tête située sensiblement suivant cet axe et à laquelle sont fixées, et de laquelle s'étendent, plusieurs pattes constituées d'au moins un élément allongé (filiforme) présentant une première extrémité et une seconde extrémité opposée, lesdites pattes étant mobiles radialement entre un état radialement déployé et un état radialement replié dans lequel les pattes sont rapprochées de l'axe, l'élément allongé de certaines au moins de ces pattes présentant une forme repliée sur elle-même, sensiblement en boucle.

Une telle structure de filtre est divulguée dans US-A-5 344 427.

Toutefois, ce filtre, comme ceux susmentionnés, présente encore des imperfections quant à la perméabilité de sa tête au regard de la circulation du sang.

Or, il s'agit là d'un problème critique, dès lors que le filtre doit être apte à capturer et à retenir des caillots sanguins d'au moins une certaine taille, mais il ne doit pas être excessivement perturbant au regard de la circulation du sang et du vaisseau dans lequel il est implanté.

Ces deux contraintes sont difficiles à satisfaire ensemble.

On doit également tenir compte de la résistance mécanique du filtre, de sa capacité à être implanté à partir d'un tube (cathéter) de petit diamètre, et de sa fiabilité.

La solution de l'invention consiste, sur un filtre du type de US-A-5 133 733 OU FR-A-2 713 081 (pages 4 à 8), à situer au moins l'une desdites extrémités de l'(des) élément(s) allongé(s) constituant les pattes, à l'écart de l'intérieur de la tête du filtre.

De cette manière, il doit être possible de rapprocher les unes des autres les portions desdits éléments allongés fixées à la tête du filtre, en diminuant alors la dimension (en particulier radiale) de cette tête, ceci devant favoriser la perméabilité de la tête du filtre au regard de la circulation du sang.

Cette solution peut également être appliquée à un filtre sanguin ayant d'autres caractéristiques structurelles, tel que le filtre divulgué dans US-A-5 344 427.

Il s'agit d'un filtre sanguin dans lequel, en plus des caractéristiques susmentionnées,
- les pattes, constituées chacune d'un élément allongé (filiforme), présentent une longueur suivant leur allongement,
- dans leur état déployé, ces pattes sont, sur une partie de leur longueur, inclinées par rapport à l'axe du filtre pour définir ensemble une corolle axiale,
- la première extrémité des éléments allongés constituant les pattes est fixée à l'intérieur de la tête du filtre,
- et surtout, la forme repliée sur elle-même, sensiblement en boucle, de certains au moins desdits éléments allongés est en outre pliée sensiblement en épingle à cheveux, de sorte que les pattes considérées présentent, autour de la corolle, un patin de centrage sensiblement parallèle à l'axe du filtre, à la suite de leur dite partie inclinée.

A la lecture de US-A-5 344 427 (qui est introduit dans la présente description par référence, comme les autres brevets cités), on notera l'importance de ces patins de centrage (appelés épingle : "harpin" ou "pin").

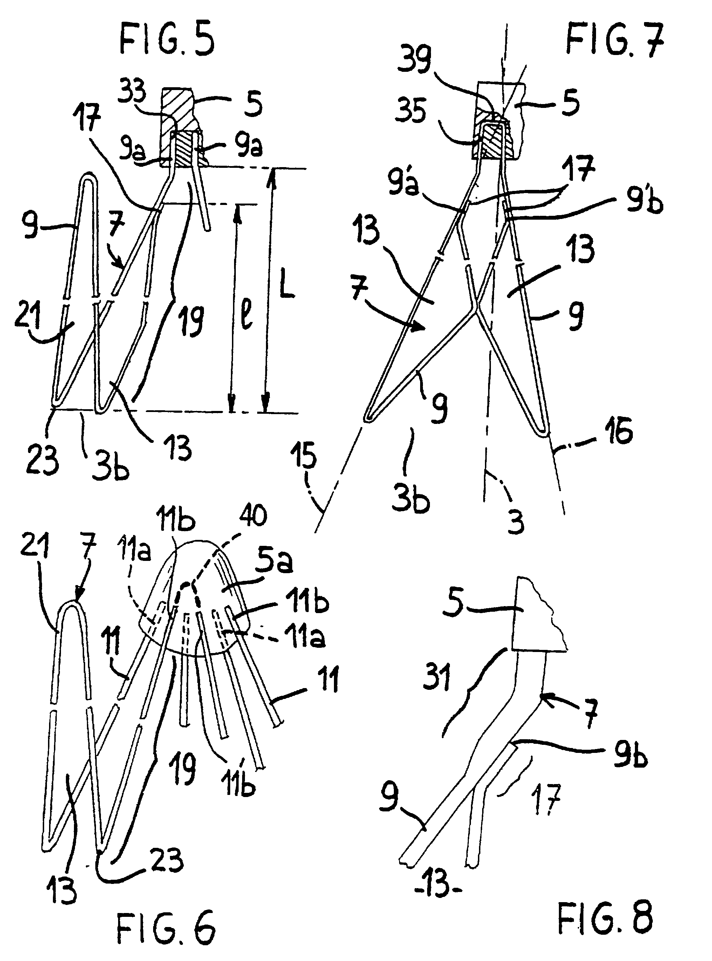

On notera également, en particulier au vu des figures 4, 5 et 15, que les deux extrémités opposées de chacun des éléments allongés constituant les pattes sont prévues pour être fixées à l'intérieur de la tête du filtre, ce qui, bien entendu, oblige à dimensionner en conséquence cette tête.

Au contraire, dans l'invention, on conseille donc qu'une des extrémités de certains au moins de ces éléments allongés soit située à l'écart de l'intérieur de la tête du filtre.

Un problème corollaire que résout l'invention concerne la manière dont on va disposer cette extrémité d'élément allongé située à l'écart de l'intérieur de la tête du filtre.

En effet, on a déjà indiqué ci-avant que le filtre doit avoir une capacité suffisante de filtration, sans toutefois être trop encombrant ni trop perturbant, tout en demeurant mécaniquement résistant.

Aussi, une caractéristique complémentaire de l'invention prévoit-elle que ladite seconde extrémité d'un élément allongé de patte soit fixée à l'élément allongé lui-même, pour fermer ladite boucle.

Les pattes considérées ne seront alors fixées à la tête du filtre que par un seul tronçon d'élément allongé, alors qu'elles le sont par deux tronçons, notamment dans US-A-5 344 427 ou FR-A-2 713 081.

Il pourrait malgré tout être considéré que cette liaison par un seul brin entre les pattes et la tête affaiblit trop la tenue mécanique des pattes et/ou leur résistance radiale à la réaction exercée par les parois du vaisseau, en situation implantée du filtre.

Aussi, une caractéristique complémentaire de l'invention prévoit-elle la possibilité que cette seconde extrémité d'élément(s) allongé(s) soit malgré tout fixée à la tête du filtre, mais alors à l'extérieur de cette tête.

On pourrait ainsi prévoir de fixer la moitié des éléments allongés (fils métalliques, par exemple) à l'intérieur de la tête et l'autre moitié, à l'extérieur de cette tête.

L'avantage de cette solution par rapport à la précédente serait de ne pas affaiblir l'assemblage des pattes à la tête du filtre, tout en augmentant la distance séparant deux brins consécutifs.

Si l'on revient toutefois à la solution première de fixer la seconde extrémité d'un élément allongé déterminé à lui-même, pour fermer la boucle, on notera l'avantage qu'il y a à situer cette fixation de l'élément à lui-même sur la portion des pattes qui se déploie pour former la corolle, ceci sur un filtre à patin de centrage du type de US-A-5 344 427.

En effet, on pourra ainsi bénéficier de la capacité de filtration de deux brins de fil, au lieu d'un, non seulement à l'endroit du patin, mais également sur ladite partie en corolle.

En relation avec cela, et à nouveau pour favoriser la tenue mécanique du filtre, une autre caractéristique de l'invention conseille de disposer cette fixation de l'élément allongé sur lui-même plus près de la tête du filtre que du patin de centrage de la patte considérée.

En complément ou en alternative, il pourra aussi s'avérer nécessaire d'augmenter la section de l'élément allongé (par exemple d'augmenter le diamètre du ou des fil(s) rond(s) constituant les pattes) à proximité de la tête du filtre et/ou sur la partie de l'élément allongé située hors de la boucle, c'est-à-dire donc sur la partie où la patte considérée ne présente qu'une structure "simple", à différencier de la structure "double" à l'endroit de la boucle.

Une description encore plus détaillée de l'invention va maintenant être fournie, en référence aux dessins annexés, dans lesquels :
- la figure 1 est une vue schématique, de face, du filtre de l'invention avec ses pattes représentées dans leur position radialement déployée ;
- la figure 2 montre le filtre de la figure 1, en position radialement resserrée de ses pattes ;
- la figure 3 montre une possible réalisation d'un tronçon d'élément allongé formant les pattes ;
- la figure 4 montre une variante de réalisation du tronçon de la figure 3 ;
- la figure 5 montre l'une des pattes du filtre de la figure 1, y compris sa fixation à la tête ;
- la figure 6 montre une vue partielle schématique d'une variante de fixation des pattes à la tête du filtre ;
- la figure 7 montre elle aussi (avec arrachement partiel comme la figure 5), une autre manière de réaliser les pattes du filtre et de les lier à la tête ;
- la figure 8 montre, en vue agrandie, un détail de la figure 5 ;
- la figure 9 montre une portion de filtre, pour la réalisation d'un filtre temporaire ;
- la figure 10 montre également une portion du filtre, pour réaliser un filtre repositionnable (voire retirable) ;
- la figure 11 montré le filtre de la figure 1 supposé en position déployée à l'intérieur d'un vaisseau sanguin ;
- et la figure 12 montre une autre variante de réalisation d'un filtre conforme à l'invention, en vue extérieure de face.

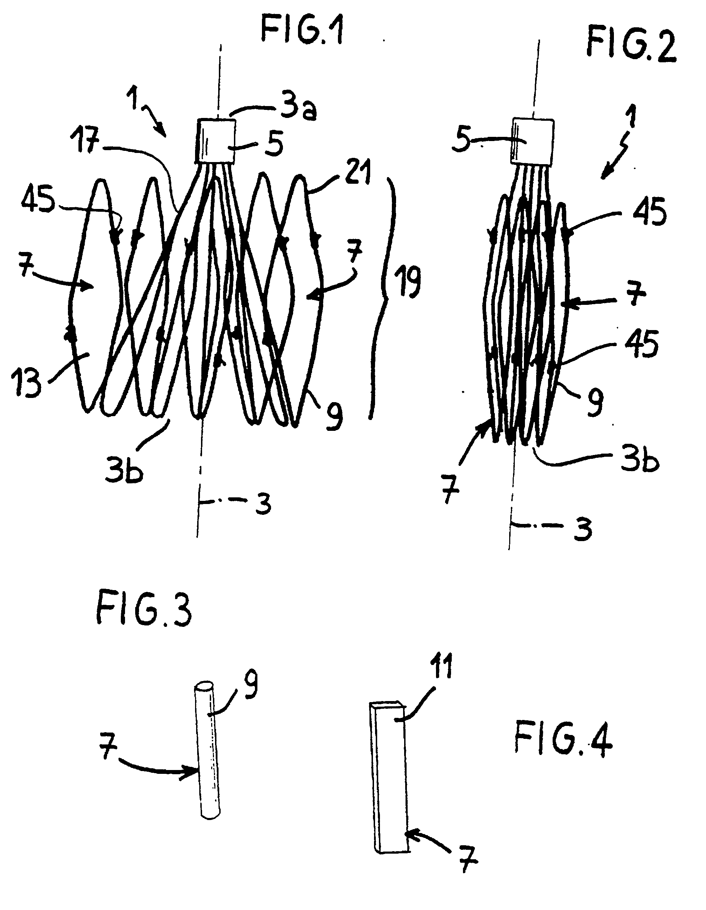

Sur la figure 1 tout d'abord, on voit illustré un filtre sanguin 1, conforme à l'invention qui doit piéger et retenir des caillots de sang de taille supérieure ou égale à 5 mm ou 6 mm (à 10 % prés).

Ce filtre présente un axe général 3, une extrémité axiale proximale 3a et une extrémité axiale distale 3b.

A son extrémité proximale, et sensiblement suivant l'axe 3, le filtre comprend une tête 5. A cette tête, sont fixées une série de pattes flexibles allongées 7, par exemple au nombre de quatre, réparties angulairement de manière sensiblement régulière autour de l'axe 3, de telle manière que ces pattes se déploient élastiquement d'abord sensiblement suivant une corolle axiale conique à partir de la tête, dans leur position radialement déployée de la figure 1.

Typiquement, il s'agira de la position de repos non contrainte des pattes.

Sur la figure 2, on voit que ces mêmes pattes 7 peuvent également occuper une position radialement resserrée dans laquelle elles sont rapprochées de l'axe 3, vis-à-vis duquel elles s'étendent ici sensiblement parallèlement.

Typiquement, il pourra s'agir là de la position radialement contrainte desdites pattes, position qu'on leur fera adopter en particulier pour mettre en place le filtre à l'intérieur du vaisseau, comme cela est connu (voir si nécessaire brevets précités, et notamment US-A-5 344 427 ou US-A-5 324 304).

Comme illustré schématiquement sur la figure 3, tout ou partie des pattes 7 peut être réalisé à partir d'un fil rond ou sensiblement rond (oval, ovoïde, ...) 9, tel en particulier un fil métallique en acier inoxydable (par exemple référencé AFNOR K13 C20 N16 Ne15, commercialisé sous la marque "PHYNOX", avec un diamètre de fil pouvant être compris entre deux et quatre dixièmes de millimètre environ). Le même métal peut être utilisé pour la tête.

Comme illustré sur la figure 4, les pattes 7 pourraient également être réalisées à partir d'une fine plaque de métal (par exemple la même matière que ci-dessus) 11, même si a priori, on conseille l'utilisation d'un fil rond.

On peut également prévoir l'utilisation d'une matière plastique, à la place du métal, la matière retenue étant de toute façon biocompatible.

A nouveau sur la figure 1, mais également sur les figures 5, 6 et 7, on notera que tout ou partie des pattes 7 est conformé en boucle, par repliement sur eux-mêmes des éléments allongés 9 ou 11.

Sur la figure 7, il s'agit de boucles 13 de forme ovoïde ou plus ou moins en trapèze allongé, chaque patte s'étendant, dans son état radialement déployé, suivant une direction (telle que 15, 16) inclinée par rapport à l'axe 3, tout en étant concourante entre elles et vis-à-vis dudit axe 3, du côté de l'extrémité proximale 3a.

Sur les figures 1, 2, 5 et 7, la boucle formée par l'un des fils 9 replié est une boucle fermée. Une vue agrandie de cette "fermeture" est illustrée sur la figure 8. On peut ainsi constater que l'extrémité 9b d'un fil 9 considéré est, à l'écart de la tête, fixée au fil lui-même (par exemple par soudage, collage,...), après que l'on ait replié en boucle ledit fil, pour former l'espace 13 fermé.

Avantageusement, la zone 17 de fixation du fil sur lui-même sera située entre l'extrémité distale 3b et la tête 5, donc sur la partie en corolle des pattes, en position radialement déployée du filtre.

On conseille même, comme montré clairement sur les figures 1, 5 et 7, que cette zone 17 soit située plus près de la tête 5 que de l'extrémité distale 3b.

Ainsi, on voit sur la figure 5 que si une distance axiale L sépare la tête 5 de l'extrémité 3b, la distance axiale l entre cette même extrémité distale 3b et la zone 17 pourra être comprise entre environ les deux tiers et les quatre cinquième de la longueur L.

Sur la figure 7, on aura en outre certainement noté que les pattes 7 s'étendent essentiellement suivant une direction rectiligne ou éventuellement légèrement courbe, avec une concavité dirigée vers l'axe 3 (directions 15 et 16).

Par contre, on remarquera sur les figures 1, 5 et 6 que les pattes 7 se prolongent par ce que l'on dénommera un "patin de centrage" 21 au-delà et autour de leur portion 19 en corolle.

Par comparaison avec la figure 7, chaque patin 21 prolonge ainsi les pattes considérées du filtre, au-delà de l'extrémité distale 3b, sans toutefois nécessairement allonger la longueur axiale L desdites pattes, puisque les patins 21 reviennent en arrière vers la tête 5 (ici commune à toutes les pattes), les deux brins des éléments allongés 9 ou 11 formant les pattes étant pour cela chacun pliés plus ou moins en épingle à cheveux, en 23.

Avec ces patins 21, la boucle 13 est donc pliée sensiblement en "V" (dans l'état radialement déployé du filtre).

Pour plus de détails, on se reportera à US-A-5 344 427 (colonnes 3 et 4).

Quoi qu'il en soit, avec une telle boucle 13 fermée en 17, la fixation des pattes considérées à la tête 5 ne s'effectuera individuellement que par un seul brin d'élément allongé 9 ou 11.

De ce fait, on peut augmenter le diamètre ou la section droite de la portion de patte située entre la zone de fermeture 17 de la boucle 13 et la zone de fixation à la tête 5 (tronçon repéré 31 sur la figure 8).

Quant à la fixation des pattes à la tête commune 5, elle pourra notamment s'effectuer de l'une des manières divulguée dans US-A-5 344 427.

Brièvement, il est tout d'abord possible de fixer l'extrémité 9a de chaque élément allongé considéré (opposé à l'extrémité précité 9b) à l'intérieur de la tête dans un passage 33 parallèle à l'axe 3, comme illustré sur la figure 5.

Ainsi, une succession de passages 33 pourront être répartis autour de cet axe à l'intérieur de la tête du filtre, pour recevoir chacune des pattes 7, la patte étant engagée étroitement dans l'espace 33 correspondant où elle est ensuite fixée (de préférence soudée ou collée).

En variante, comme illustré sur la figure 7, on envisage aussi la possibilité d'utiliser un élément allongé, tel qu'un fil métallique 9, plus long que celui de la figure 5 et de faire passer une portion sensiblement centrale 39 dudit fil à l'intérieur d'un passage 35 en "U" de la tête 5, les deux extrémités libres 9'a, 9'b du fil étant ensuite chacune fixées en 17 sur le fil lui-même, pour former les boucles souhaitées.

Eventuellement, l'extrémité 9'b du fil pourrait même se prolonger pour passer encore une ou plusieurs fois à l'intérieur de la tête et développer ainsi les pattes sur toute la périphérie de cette tête, à partir d'un seul fil, avec une succession de passages en "U" 35 se rejoignant éventuellement à l'intérieur de la tête.

Sur les figures 6, 9 et 10, est illustrée une autre manière de doubler les pattes et de les fixer à la tête du filtre.

En l'espèce, comme on le voit plus clairement sur la figure 6, les deux extrémités opposées 11a, 11b d'un même élément allongé 11 sont ramenées jusqu'à la tête 5, où elles sont fixées respectivement à l'intérieur et à l'extérieur de ladite tête, après que l'on ait défini la patte 7 bouclée avec éventuellement son patin 21.

La fixation de l'extrémité 11a à l'intérieur de la tête peut s'opérer comme sur la figure 5, dans un passage parallèle à l'axe 3.

A l'extérieur, la portion terminale opposée 11b du même élément est plaquée contre la surface 5a de la tête, à laquelle cette portion terminale est fixée par tout moyen approprié, tel que soudage ou collage.

Ainsi, la boucle réalisée ne sera pas tout à fait fermée, une épaisseur de la tête séparant les deux extrémités 11a, 11b, lesquelles pourront toutefois être plus ou moins rapprochées, voire se chevaucher.

A noter que, même si un élément 11 se prolongeait par un tronçon (pointillés 40 sur la figure 6) pour relier entre elles deux extrémités successives (telles que 11b et 11'b) de deux éléments 11 adjacents fixés à la tête, on considérerait quand même qu'il s'agit d'une fixation à la tête du filtre par une "extrémité" d'élément allongé. La même considération peut être appliquée à la figure 7, au regard du tronçon de fil 39.

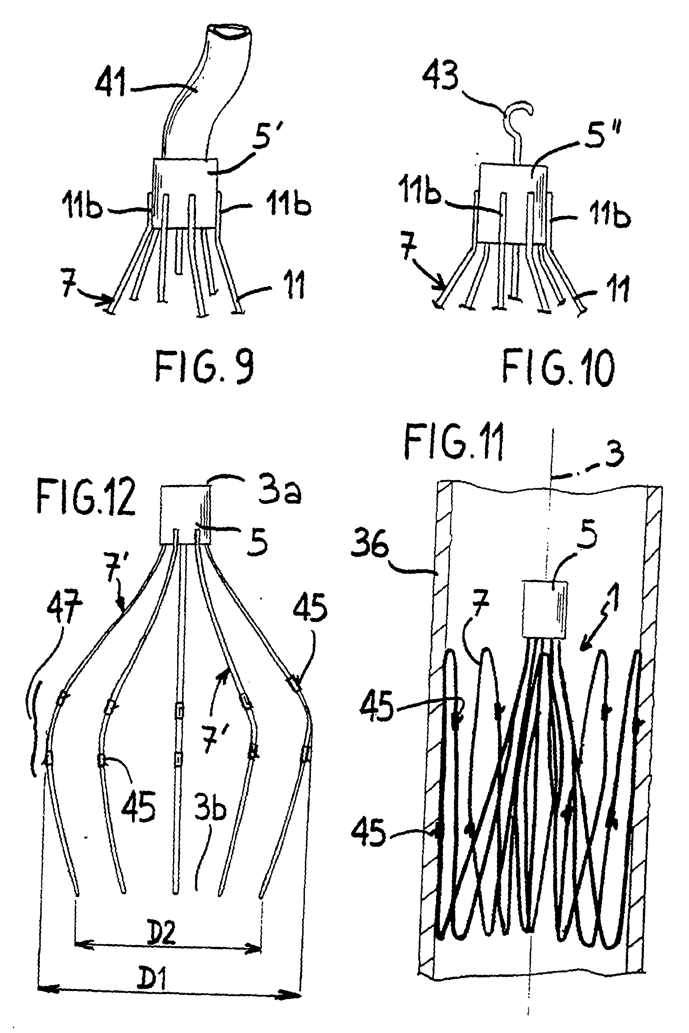

Sur les figures 9 et 10, la fixation des pattes est la même que sur la figure 6.

Mais ces deux figures présentent surtout un intérêt au regard du moyen complémentaire dont leur tête (respectivement 5' et 5") est pourvue, pour autoriser ou faciliter le déplacement du filtre à l'intérieur du vaisseau qui doit le recevoir, voire le retrait dudit filtre après une certaine période d'implantation.

Ainsi, sur la figure 9, la tête du filtre est-elle raccordée, à l'opposé des pattes, à un cathéter ou une tige souple 41, typiquement utilisé pour la manoeuvre des filtres temporaires, comme cela est divulgué notamment dans FR-A-2 713 081 ou US-A-5 300 086, la souplesse de la tubulure 41 étant adaptée pour qu'elle passe sans dommage à l'intérieur de la voie d'accès vasculaire, depuis l'extérieur du corps du patient jusqu'au vaisseau récepteur 36 du filtre (voir figure 11).

Sur la figure 10, la tête 5" comporte un crochet 43 à la place de la tubulure 41, comme cela est prévu dans US-A-5 324 304 (moyen dénommé crochet de retrait, "retrieval hook"), le crochet 43 étant bien entendu à utiliser en combinaison avec les moyens complémentaires décrits dans ce brevet.

Ainsi, le filtre de l'invention peut être un filtre temporaire, ou du moins un filtre déplaçable à l'intérieur du vaisseau qui le reçoit.

S'il s'agit malgré tout d'un filtre "définitif", il sera alors avantageusement (de manière conventionnelle) pourvu de moyens de fixation à la paroi dudit vaisseau.

A cet effet, les parois des pattes pourraient être traitées chimiquement pour favoriser localement leur adhésion à cette paroi.

En complément ou en alternative, des crochets peuvent être prévus sur les pattes, comme illustré sur les figures 1, 2 et 12.

Ces crochets, repérés 45, peuvent être dirigés alternativement en direction de l'extrémité proximale 3a et de l'extrémité distale 3b, pour assurer la retenue du filtre dans les deux sens, sensiblement suivant l'axe 3.

La réalisation et la fixation des crochets 45 peuvent être obtenues de la manière divulguée dans US-A-5 344 427.

Ces crochets ont été disposés sur les éléments allongés constituant les pattes du filtre et, plus précisément, à l'endroit d'une portion venant étroitement en contact de la paroi du vaisseau, dans l'état radialement déployé dudit filtre.

Ainsi, on trouve ces crochets à l'endroit des patins 21, sur les figures 1 et 2, et sur la portion la plus ventrue (donc de plus grand diamètre) 47 sur la figure 12.

Sur cette figure, on remarquera la forme cintrée vers l'extérieur des pattes 7', en position radialement déployée du filtre, lesdites pattes présentant donc une concavité interne.

A noter que les pattes 7' peuvent être réalisées en fil (9) ou en plaque étroite (11), et avec ou sans boucle.

En l'espèce, les pattes sont en fils métalliques légèrement courbées en arc, avec un diamètre intermédiaire des pattes du filtre en position radialement déployée, D1, qui est supérieur au diamètre distal D2 d'ouverture de ces mêmes pattes en 3b, lequel diamètre est lui-même supérieur au diamètre desdites pattes à l'extrémité proximale 3a, là où les pattes sont fixées à la tête 5, en l'espèce de la manière représentée sur la figure 6, c'est-à-dire pour certaines à l'intérieur et, pour d'autres, à l'extérieur de la tête 5, avec ici une alternance d'une patte sur deux.

Il doit toutefois être clair que les pattes 7' pourraient être des pattes double et correspondre notamment à celles des figures 5, 6 ou 7.

A noter que cette forme des pattes "en arc" bombé vers l'extérieur doit intrinsèquement favoriser la perméabilité de la tête du filtre. L'intérêt est renforcé par le fait que le cône sera d'autant plus sollicité à s'ouvrir à son sommet que le diamètre du vaisseau d'implantation (veine cave) sera petit, contrecarrant ainsi le fait qu'un filtre est généralement d'autant plus "thrombogène" qu'il est placé dans un vaisseau de petit diamètre.

## Revendications

1. Filtre sanguin (1) destiné à être positionné dans un vaisseau sanguin (36) pour y piéger des caillots de sang, le filtre présentant un axe (3) et comprenant une tête (5, 5a, 5', 5") située sensiblement suivant cet axe, et à laquelle sont fixées, et de laquelle s'étendent, plusieurs pattes (7, 7') constituées d'au moins un élément allongé (9, 11) présentant deux extrémités opposées (9a, 9b ; 11a, 11b ; 9'a, 9'b), lesdites pattes étant mobiles radialement entre un état radialement déployé et un état radialement replié où les pattes sont rapprochées de l'axe,
**caractérisé en ce que** l'une au moins des extrémités (9b, 9'a, 9'b, 11b) du ou des éléments allongés (9, 11) constituant les pattes est située à l'écart de l'intérieur de la tête (5, 5a, 5', 5") du filtre.

2. Filtre sanguin selon la revendication 1 **caracterisé en ce que** l'élément allongé de certaines au moins des pattes (7, 7') présente une forme repliée sur elle-même, sensiblement en boucle (13).

3. Filtre sanguin (1) destiné à être positionné à l'intérieur d'un vaisseau sanguin (36), pour y piéger des caillots, lequel filtre :
- présente un axe (3),
- comprend une tête (5, 5a, 5', 5") située sensiblement suivant cet axe et à laquelle sont fixées, et de laquelle s'étendent, plusieurs pattes (7) constituées chacune d'un élément allongé (9, 11) présentant une première extrémité (9a, 11a) fixée à l'intérieur de la tête et une seconde extrémité opposée (9b, 11b),
- les pattes sont mobiles radialement entre un état radialement déployé et un état radialement replié où elles sont rapprochées de l'axe,
- l'élément allongé (9, 11) de certaines au moins desdites pattes présente une forme repliée sur elle-même, sensiblement en boucle (13),
- dans leur état déployé, les pattes sont, sur une partie de leur longueur, inclinées par rapport à l'axe (3) du filtre, pour définir ensemble une corolle axiale,
- et la forme repliée sur elle-même, sensiblement en boucle, de certains au moins desdits éléments allongés est pliée sensiblement en épingle à cheveux, de sorte que les pattes considérées présentent, autour de la corolle, un patin (21) de centrage sensiblement parallèle à l'axe du filtre, à la suite de leur dite partie inclinée (19),
**caractérisé en ce que** ladite seconde extrémité (9b, 11b) de certains au moins des éléments allongés (9, 11) constituant les pattes est située à l'écart de l'intérieur de la tête (5, 5a, 5', 5") du filtre.

4. Filtre selon la revendication 3, **caractérisé en ce que** ladite seconde extrémité (9b) d'un élément allongé (9, 11) d'une patte (7) déterminée du filtre, non située à l'intérieur de la tête (5) de ce filtre, est fixée à l'élément allongé (9) lui-même, pour fermer ladite boucle (13).

5. Filtre selon la revendication 4, **caractérisé en ce que** la fixation de ladite seconde extrémité (9b) d'un élément allongé (9, 11) à l'élément lui-même, pour former la boucle, est située sur la partie en corolle, en position déployée des pattes (7).

6. Filtre selon la revendication 4 ou la revendication 5, **caractérisé en ce que** la fixation de ladite seconde extrémité (9b) d'un élément allongé (9, 11) à l'élément lui-même pour fermer la boucle (13), est située plus près de la tête (5) du filtre que du patin (21) de la patte considérée.

7. Filtre selon l'une quelconque des revendications 4 à 6, **caractérisé en ce que**, entre la tête (5) du filtre et l'endroit (17) de fixation de l'élément allongé (9, 11) sur lui-même pour fermer ladite boucle (13), la section de l'élément considéré est supérieure à ce qu'elle est à l'endroit de ladite boucle.

8. Filtre selon la revendication 1 ou la revendication 3, **caractérisé en ce que** l'une des extrémités (11b) de certains au moins des éléments (9, 11) allongés est fixée à la tête (5, 5a, 5', 5''), à l'extérieur de cette tête.

9. Filtre selon la revendication 1 ou la revendication 8 , **caractérisé en ce que** :
- le filtre présente, axialement, une extrémité proximale (3a) du côté de sa tête (5), et une extrémité distale (3b),
- dans un état non contraint, les éléments allongés (9, 11) constituant les pattes sont cintrés suivant leur direction d'allongement, avec une concavité dirigée vers l'axe (3) du filtre,
- et le diamètre (D2) du cercle défini par les pattes à ladite extrémité distale (3b) est inférieur à celui (D1) défini par les pattes en une zone intermédiaire située entre lesdites extrémités proximale et distale (3a, 3b).

## Claims

1. A blood filter (1) adapted to be disposed in a blood vessel (36) for retaining blood clots, the blood filter having an axis (3) and comprising a head (5, 5a, 5', 5") disposed substantially on the axis and to which are fixed, and from which extend, a plurality of legs (7, 7') comprising at least one elongated element (9, 11) having two opposed ends (9a, 9b; 11a, 11b; 9'a, 9'b), the legs being radially movable between a radially expanded state and a radially restrained state in which the legs are brought close to the axis, **characterised in that** at least one of the ends (9b, 9'a, 9'b, 11b) of the elongated element(s) (9, 11) is disposed apart from inside the head (5, 5a, 5', 5") of the blood filter.

2. The blood filter according to claim 1, **characterised in that** the elongated element of at least some of the legs (7,7') has a shape folded back on itself, substantially as a loop (13).

3. A blood filter adapted to be disposed in a blood vessel, for retaining blood clots, in which :
- the blood filter has an axis (3),
- the blood filter comprises a head (5, 5a, 5', 5") disposed substantially on the axis and to which are fixed, and from which extend, a plurality of legs (7) each of which comprises at least one elongated element (9, 11) having a first end (9a, 11a) fixed inside the head and a second opposed end (9b, 11b),
- the legs are radially movable between a radially expanded state and a radially restrained state, in which said legs are brought close to the axis,
- the elongated element (9,11) of at least some of said legs has a shape folded back on itself, substantially as a loop (13),
- in the radially expanded state, the legs are inclined from the axis (3) along a portion of their length, for defining together an axial corolla,
- the shape folded back on itself, substantially as loop, of at least some of said elongated elements, is hairpin folded, so that said legs have, round the corolla, a centering pad (21) disposed substantially parallel to the filter axis, as an extension of said inclined portion (19),
**characterised in that** said second end (9b, 11b) of at least some of the elongated elements (9, 11) defining the legs are located apart from inside the head filter (5, 5a, 5', 5").

4. The filter according to claim 3, **characterised in that** said second end (9b) of a determined elongated element (9, 11) of one leg (7), which is not located inside the head (5) of the filter, is fixed to said determined elongated element itself (9), for closing the loop (13).

5. The filter according to claim 4, **characterised in that** the fixation of the second end (9b) of said determined elongated element (9,11) to itself is disposed on the corolla, in the radially expanded state of the legs (7).

6. The filter according to claim 4 or claim 5, **characterised in that** the fixation of said second end (9b) of a determined elongated element (9,11) to itself, for closing the loop (13), is disposed closer to the head filter (5) than the centering pad (21) of the corresponding leg.

7. The filter according to anyone of claims 4 to 6, **characterised in that**, between the head filter (5) and the zone (17) of fixation of said elongated element (9, 11) to itself for closing the loop (13), the section of said determined elongated element is larger than along the loop.

8. The filter according to claim 1 or claim 3, **characterised in that** one of the ends (11b) of at least some of the elongated elements (9, 11) is connected to the head (5, 5a, 5', 5"), outside said head.

9. The filter according to claim 1 or claim 8, **characterised in that**:
- axially, the filter has a proximal end (3a) close to the head (5) and an distal end (3b),
- in a radially non-restrained state, the elongated elements (9, 11) defining the legs are curved along their length and have a concavity directed to the axis (3) of the filter,
- and, at the distal end, the legs define a circle having a diameter (D2) which is narrower than the diameter (D1) which is defined by said legs at a zone intermediate between said proximal and distal ends (3a, 3b).

## Patentansprüche

1. Blutfilter (1) zur Anordnung in einem Blutgefäß (36), um dort Blutgerinnsel zu sammeln, wobei der Filter eine Achse (3) besitzt und einen Kopf (5, 5a, 5', 5") hat, der im Wesentlichen in dieser Achse angeordnet ist und an dem mehrere Füße (7, 7') befestigt sind, die sich von ihm weg erstrecken, die aus wenigstens einem länglichen Element (9, 11) bestehen, das zwei gegenüber liegende Enden (9a, 9b; 11a, 11b; 9'a, 9'b) besitzt, wobei die Füße radial zwischen einem radial entfalteten Zustand und einem radial zusammengefalteten Zustand, in dem sie an die Achse angenähert sind, beweglich sind, **dadurch gekennzeichnet, dass** wenigstens eines der Enden (9b, 9'a, 9'b, 11) des länglichen Elements oder der länglichen Elemente (9, 11), die die Füße bilden, außerhalb des Inneren des Kopfs (5, 5a, 5', 5") des Filters liegt.

2. Blutfilter nach Anspruch 1, **dadurch gekennzeichnet, dass** das längliche Element von wenigstens einigen Füßen (7, 7') eine im Wesentlichen als Schleife (13) zu sich umgebogene Form darbietet.

3. Blutfilter (1) zur Anordnung in einem Blutgefäß (36), um dort Blutgerinnsel zu sammeln, wobei der Filter:
- eine Achse (3) besitzt,
- einen Kopf (5, 5a, 5', 5") hat, der im Wesentlichen in dieser Achse liegt und an dem mehrere Füße (7) befestigt sind, die sich von ihm weg erstrecken und jeweils aus einem länglichen Element (9, 11) bestehen, das ein erstes Ende (9a, 11a) aufweist, das in dem Kopf befestigt ist, sowie ein gegenüber liegendes zweites Ende,
- wobei die Füße radial zwischen einem radial entfalteten und einem radial zusammengefalteten Zustand, in dem sie an die Achse angenähert sind, beweglich sind,
- wobei das längliche Element (9, 11) wenigstens von einigen Füßen im Wesentlichen eine als Schleife (13) zu sich umgebogene Form darbietet,
- wobei die Füße in ihrem entfalteten Zustand entlang eines Teils ihrer Länge gegenüber der Achse (3) des Filters geneigt sind, um gemeinsam eine axiale Krone zu bilden,
- und wobei die auf sich im Wesentlichen als Schleife umgebogene Form im Wesentlichen als Haarnadel gebogen ist, so dass die betreffenden Füße nach ihrem geneigten Teil (19) um die Krone einen Zentrierschuh (21) bilden, der im Wesentlichen parallel zu der Achse des Filters liegt,
**dadurch gekennzeichnet, dass** das zweite Ende (9b, 11b) wenigstens einiger längliche Elemente (9, 11) außerhalb des Inneren des Kopfs (5, 5a, 5', 5") des Filters angeordnet ist.

4. Filter nach Anspruch 3, **dadurch gekennzeichnet, dass** das zweite Ende (9b) eines längliche Elements (9, 11) eines von dem Filter definierten Fußes (7), das nicht im Inneren des Kopfs (5) dieses Filters angeordnet ist, an dem länglichen Element (9) selbst befestigt ist, um die Schleife (13) zu schließen.

5. Filter nach Anspruch 4, **dadurch gekennzeichnet, dass** die Befestigung des zweitens Endes (9b) eines länglichen Elements (9, 11), an dem Element selbst, um die Schleife zu schließen, in der entfalteten Position der Füße (7) an dem kronenförmigen Teil angeordnet ist.

6. Filter nach Anspruch 4 oder 5, **dadurch gekennzeichnet, dass** die Befestigung des zweitens Endes (9b) eines längliche Elements (9, 11) an dem Element selbst, um die Schleife (13) zu schließen näher am Kopf (5) des Filters angeordnet ist, als an dem Gleitschuh (21) des betreffenden Fußes.

7. Filter nach einem der Ansprüche 4 bis 6, **dadurch gekennzeichnet, dass** zwischen dem Kopf (5) des Filters und dem Ort (17) der Befestigung des länglichen Elements (9 11), an sich selbst, um die Schleife (13) zu bilden, der Querschnitt des betreffenden Elements größer ist, als an dem Ort der Schleife.

8. Filter nach Anspruch 1 oder 3, **dadurch gekennzeichnet, dass** eines der Enden (11b) wenigstens einiger länglicher Elemente (9, 11) von außen an dem Kopf (5, 5a, 5', 5") befestigt ist.

9. Filter nach Anspruch 1 oder Anspruch 9. **dadurch gekennzeichnet, dass**:
- der Filter axial ein proximales Ende (3a) an der Seite seines Kopfs (5), sowie ein distales Ende (3b) besitzt,
- wobei in einem nicht zusammengezogenen Zustand die länglichen Elemente (9, 11), die die Füße bilden, in ihrer Längsrichtung mit einer zur Achse (3) des Filters ausgerichteten Konkavität gewölbt sind,
- und wobei der Durchmesser (D2) der durch die Füße am distalen Ende (3) definiert wird, kleiner ist, als derjenige (D1), der durch die Füße in einer Zwischenzone zwischen dem proximalen und dem distalen Ende (3a, 3b) definiert wird.
